# EUROPÄISCHE PATENTSCHRIFT

(11) **EP 2 220 126 B1**
(45) Veröffentlichungstag und Bekanntmachung des Hinweises auf die Patenterteilung: **16.11.2011**
(21) Anmeldenummer: 08859446.0
(22) Anmeldetag: 05.12.2008
(51) Int. Cl.: C08B 37/00

(54) **CYCLODEXTRIN-SILANKOMPLEXE**
CYCLODEXTRIN-SILANE COMPLEXES
COMPLEXE CYCLODEXTRINE-SILANE

(30) Priorität: 12.12.2007 DE 102007055776
(43) Veröffentlichungstag der Anmeldung: 25.08.2010
(73) Patentinhaber: Wacker Chemie AG, 81737 München (DE)
(72) Erfinder: AMANN, Manfred, 85235 Odelzhausen (DE); HECHT, Wolfgang, 80796 München (DE)
(74) Vertreter: Potten, Holger
(86) Internationale Anmeldenummer: PCT/EP2008/066862
(87) Internationale Veröffentlichungsnummer: WO 2009/074512

(56) Entgegenhaltungen:
- WO-A-96/31540
- JP-A- 2004 277 410
- US-A- 4 781 858
- US-A- 5 384 186
- OKUMURA H ET AL: "Preparation and characterization of inclusion complexes of poly(dimethylsiloxane)s with cyclodextrins" MACROMOLECULES 20010828 AMERICAN CHEMICAL SOCIETY US, Bd. 34, Nr. 18, 28. August 2001 (2001-08-28), Seiten 6338-6343, XP002522624 in der Anmeldung erwähnt
- FELIX KLAUS ET AL: "Tetravinyl-tetramethylcyclo-tetrasiloxane (tetravinyl D4) is a mutagen in Rat2lambdalacI fibroblasts" CARCINOGENESIS (OXFORD), Bd. 19, Nr. 2, Februar 1998 (1998-02), Seiten 315-320, XP002522625 ISSN: 0143-3334

## Beschreibung

Silane werden in vielfältiger Weise zur Funktionalisierung von Oberflächen verwendet ("Silanisierung"), seien es die molekularen Oberflächen von kleinen Molekülen, seien es die Oberflächen nanoskaliger oder mikroskaliger Strukturen oder seien es makroskopische Oberflächen. Je nach Struktur zeigen die Silane unterschiedliche chemische Stabilität und unterschiedliche Reaktivitäten, was bei der Verwendung von Silanen beachtet werden muss. So hydrolysieren beispielsweise halogenierte oder alkoxylierte Silane in Gegenwart von Wasser. Die Instabilität der Silane schränkt ihre Verwendbarkeit ein. Ebenso schränken die physikalischen Eigenschaften der Silane ihre Verwendungsmöglichkeiten ein. Silane liegen üblicherweise in flüssiger Form vor, häufig sind sie bei Raumtemperatur bereits flüchtig. Die Notwendigkeit, Silane als Flüssigkeit zu verarbeiten beschränkt ihre Verwendbarkeit in vielen technischen Anwendungen. Eine wichtige Aufgabe ist es daher, Verfahren zur Stabilisierung von Silanen zu entwickeln.

Cyclodextrine (CDs) sind ringförmige nicht reduzierende Oligosaccharide, welche aus Glucoseeinheiten bestehen. Ihre äußere Oberfläche ist hydrophil, wodurch sie sich in Wasser lösen lassen. Ihre innere Oberfläche zeigt hydrophoben Charakter. Aus diesem Grunde sind CDs in der Lage, Einschlußverbindungen mit kleineren Molekülen einzugehen.

Cyclodextrinkomplexe mit siliziumhaltigen Verbindungen werden von verschiedenen Autoren beschrieben. Diese Arbeiten beschäftigen sich dabei mit Cyclodextrin-Polymer Wechselwir-kungen, oder mit Wechselwirkungen von Cyclodextrinen mit nie-dermolekularen Siloxanen nicht aber mit Cyclodextrin Silan Wechselwirkungen.

So beschreiben Okumura et.al. (2001, Makromolecules 34, 6338-43) das Auffädeln von Cyclodextrin auf Polydimethylsiloxane. Wenz et al. beschreiben ganz allgemein (Chem. Review 2006, 106, 782 -817) die Cyclodextrin - Polymer Wechselwirkungen (Rotaxane und Polyrotaxane). Die technische Verwendung solcher Komplexe wird in WO 9631540 A1 beschrieben.

Felix, K. et al. (1996) Curr.Top.Microbiol.Immunol. 210 (Immunology of Silicones), 93-9) beschreiben Membranwechsel-wirkungen von gamma-CD Komplexen mit Octamethylcyclotetrasiloxan und anderen kleinen stabilen Siloxanen.

Aufgabe der Erfindung ist es Silane so zur Verfügung zu stellen, dass ihre technische Anwendbarkeit erhöht wird.

Die Aufgabe wird gelöst durch einen isolierten Komplex aus einem Cyclodextrin oder Cyclodextrinderivat und einem Silan gemäβ Anspruch 1.

Die in der Regel bei Raumtemperatur (20°C) und Normdruck flüssigen Silane werden im erfindungsgemäßen Komplex stabilisiert und als pulverförmiger Feststoff zur Verfügung gestellt. Diese Pulverformulierung erweitert die technische Anwendbarkeit der Silane deutlich. Als Pulver lassen sich die Silankomplexe problemlos verarbeiten und lagern.

Im Sinne der vorliegenden Erfindung sind Silane bei 20°C und Normdruck flüssige niedermolekulare Siliziumverbindungen der allgemeinen Formel (I)

YₑSiR¹₍₄₋ₑ₎ (I)

wobei
Y Wasserstoff, eine OH-Gruppe, Halogen, eine OR¹-Gruppe bedeutet,
R¹ einen gegebenenfalls Heteroatom-substituierten aliphatischen oder aromatischen Kohlenwasserstoffrest mit 1-12 Kohlenstoffatomen bedeutet und
e die Werte 1, 2, 3 und 4 annehmen kann.

Vorzugsweise handelt es sich bei dem Silan um ein hydrolyseempfindliches Silan. Besonders bevorzugt handelt es sich um ein Alkoxysilan, z.B. um ein Isooctyltriethoxysilan, ein Tetra-ethoxysilan, ein Tetra-isopropoxysilan oder ein Tetra-n-propoxysilan.

Bei dem Cyclodextrinen bzw. Cyclodextrinderivat handelt es sich um ein beliebiges Cyclodextrin bzw. Cyclodextrinderivat oder um ein Gemisch aus mindestens zwei verschiedenen Arten derartiger Cyclodextrin(derivat)e.

Vorzugsweise handelt es um ein Cyclodextrin bzw. Cyclodextrinderivat der Formel II 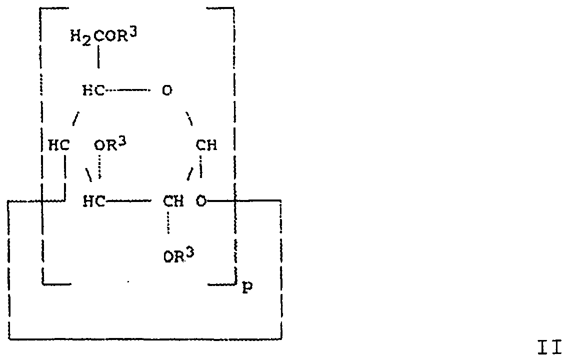 wobei R³ gleich oder verschieden sein kann und Wasserstoff oder einen gegebenenfalls Heteroatom-substituierten aliphatischen oder aromatischen Kohlenwasserstoffrest mit 1-12 Kohlenstoffatomen bedeutet und p ein ganzzahliger Wert von 6 bis 120 ist.

Beispiele für R³ sind Wasserstoffatom, Alkylrest mit 1 bis 10 Kohlenstoffatomen, Acylreste mit 1 bis 10 Kohlenstoffatomen, Glycosylrest, kationisch und anionisch geladener Rest.

Vorzugsweise handelt es sich bei R³ um Wasserstoffatom,
Methyl-, Hydroxypropyl-, Acetyl-, Carboxymethyl- oder 2-OH-3-Trimethylammoniopropylrest, wobei Methyl- und Hydroxypropylreste besonders bevorzugt sind.

Falls alle R³ Wasserstoff bedeuten, handelt es sich bei den Verbindungen der Formel (II) um native unmodifizierte Cyclodextrine. Cyclodextrine sind nach außen hydrophil, wodurch sie sich sehr leicht in Wasser lösen.

Falls mindestens ein R³ eine Bedeutung verschieden von Wasserstoff hat, handelt es sich bei den Verbindungen der Formel (II) um Cyclodextrinderivate. Mit der Anzahl und Art der derivatisierten Stellen im Cyclodextrin wird unter anderem das hydrophile Verhalten der Cyclodextrinderivate bestimmt.

Bevorzugt ist p 6, 7 oder 8 insbesondere bevorzugt 8.

Besonders bevorzugt handelt es sich bei dem Cyclodextrin im erfindungsgemäßen Komplex somit um gamma-Cyclodextrin oder um ein Derivat des gamma-Cyclodextrins.

Die Cyclodextrine bzw. Cyclodextrinderivate sind handelsübliche Produkte bzw. können nach in der Chemie bzw. Biotechnologie üblichen Verfahren hergestellt werden.

Native Cyclodextrine (alpha-, beta- und gamma- CD) und derivatisierte Cyclodextrine (z.B. methyliert, hydroxypropyliert) können beispielsweise von der Wacker Chemie AG bezogen werden.

Die Erfindung betrifft ferner die Herstellung der erfindungsgemäßen Komplexe.

Die Herstellung der Komplexe erfolgt dadurch, dass das zu komplexierende Silan und das Cyclodextrin miteinander über einen Zeitraum von 1 min bis 24 Stunden in Kontakt gebracht werden , bevorzugt 0,5 bis 5 h, besonders bevorzugt 1h und der gebildete Komplex anschließend sofort von einem ggf. vorhandenen Lösungsmittel abgetrennt wird.

Bei dem erfindungsgemäßen Verfahren werden Cyclodextrine bzw. Cyclodextrinderivate und Silane vorzugsweise im Molverhältnis von 10:1 bis 1:10, besonders bevorzugt 3:1 bis 1:3, insbesondere bevorzugt 1:1, eingesetzt.

Bei dem erfindungsgemäßen Verfahren kann das in Kontakt bringen der beiden Edukte auf beliebige Art und Weise erfolgen. Dabei werden die Cyclodextrine bzw. Cyclodextrinderivate und die Silane möglichst innig in Kontakt gebracht, beispielsweise durch starkes Verrühren, Schütteln oder Verkneten.

Das in Kontakt bringen erfolgt vorzugsweise über einen Zeitraum von 1 min bis 24 Stunden, bevorzugt 0,5 bis 5 h, besonders bevorzugt 1h.

Falls erwünscht, kann zusätzlich zu Cyclodextrin(derivat) und Silan Lösungsmittel eingesetzt werden, wobei die Bezeichnung Lösungsmittel nicht bedeutet, dass sich alle Reaktionskomponenten in diesem lösen müssen. So können Lösungsmittel verwendet werden, in denen sich sowohl Cyclodextrin(derivat) als auch Silan ganz oder teilweise lösen, wie auch Lösungsmittel, in denen sich ausschließlich Cyclodextrin(derivat) oder Silan ganz oder teilweise löst.

Bei dem gegebenenfalls eingesetzten Lösungsmittel handelt es sich vorzugsweise um Wasser, polare organische Lösungsmittel, wie Alkohole (z.B. Methanol, Ethanol, Propanol, Isopropanol und Butanol), Aceton, Tetrahydrofuran oder Dimethylsulfoxid, apolare organische Lösungsmittel, wie Acetonitril, Chloroform, Diethylether, Ethylacetat, p-Xylol und Alkane sowie deren Gemische.

Bevorzugt handelt es sich bei dem gegebenenfalls eingesetzten Lösungsmittel um Wasser.

Es ist wesentlich für das erfindungsgemäße Verfahren, dass bei Anwesenheit eines Lösungsmittels dieses unmittelbar anschließend an die Komplexierung mittels in Kontakt bringen von dem gebildeten Komplex entfernt wird.

Die Temperatur kann bei dem erfindungsgemäßen Verfahren über einen weiten Bereich variiert werden und hängt wesentlich nur von der Stabilität des Silans und des verwendeten Cyclodextrin(derivat)s ab. Das erfindungsgemäße Verfahren wird bei einer Temperatur von vorzugsweise 5 bis 95°C, besonders bevorzugt 30 bis 70°C, und bevorzugt bei dem Druck der umgebenden Atmosphäre, d.h. einem Druck zwischen 900 und 1100 hPa, durchgeführt.

Wird das erfindungsgemäße Verfahren in Anwesenheit von Lösungsmittel, insbesondere Wasser, durchgeführt, so werden erfindungsgemäße Cyclodextrin- Silankomplexe erhalten, die abhängig von der Art des Silans und des Cyclodextrin(derivat)s ganz oder teilweise im verwendeten Lösungsmittel gelöst sind. Durch Entfernen des Lösungsmittels, beispielsweise durch Temperaturbehandlung, Destillation, Einrotieren am Rotationsverdampfer, Sprühtrocknen oder Lyophilisieren können die erfindungsgemäßen Cyclodextrin- Silankomplexe isoliert werden.

Das erfindungsgemäße Verfahren hat den Vorteil, daß die erfindungsgemäßen isolierten Cyclodextrin/Silankomplexe auf einfache Art und Weise erhalten werden.

Die Erfindung betrifft ferner die Verwendung der erfindungsgemäßen Komplexe.

Der erfindungsgemäße Komplex eignet sich für verschiedene Anwendungen z.B. zur Kühlung, welche durch die Verdampfung von Ethanol erzielt werden kann.

Eine weitere Verwendung solcher Komplexe ist die Bereitstellung von Substrat für Enzyme, welche z.B. Ethanol als

Substrat verwenden können wie z.B. Alkohol spezifische Oxidasen oder Dehydrogenasen. Durch die Einwirkung von Alkoholoxidasen kann Wasserstoffperoxid gebildet werden, welches gegebenenfalls seine erwünschte Wirkung z. B. als weiteres Enzymsubstrat oder als Bleichchemikalie entfalten kann. Dies kann vorteilhaft fürt die Entwicklung von Testverfahren ausgenützt werden.

Der Komplex eignet sich ferner als Reservoir zur gezielten Freisetzung von labilen Bestandteilen des komplexierten Silans. So ist Tetraethoxysilan beispielsweise eine Speicherverbindung für vier Moleküle Ethanol.

Komplexe aus einem Silan mit einem Cyclodextrin können somit zur kontrollierten Freisetzung der Silanreste verwendet werden, z.B. als Slow Release Präparation von hydrolysierbaren Gruppen aus Silanen (Ethanol, Methanol, Isopropanol).

So lässt sich die Biocide Wirkung von Ethanol, Isopropanol und n-Propanol bei Zutritt von Feuchtigkeit zu den entsprechenden Cyclodextrinkomplexen aus Tetraethoxysilan, Tetra-isopropoxysilan oder Tetra-n-propoxysilan nutzen. Ebenso möglich ist die vorteilhafte Verwendung der bei Hydrolyse freigesetzten Kieselsäure, welche z.B. zum Aufbau von Silika basierten Nanostrukturen in geringer Konzentration benötigt wird.

Bei der Verwendung der erfindungsgemäßen Komplexe zur gezielten Freisetzung von labilen Bestandteilen des komplexierten Silans entstehen nur umweltverträgliche toxikologisch unbedenkliche Produkte (Cyclodextrine, Kieselsäure, Alkohol).

Komplexe aus CD und hydrophoben Silanen (z.B. Isooctyltriethoxysilan) eignet sich beispielsweise zur Hydrophobierung von Trockenmörteln.

Die folgenden Beispiele dienen der weiteren Erläuterung der Erfindung:
Beispiel 1: Herstellung eines Komplexes aus gamma-Cyclodextrin und Tetraethoxysilan
   8,0 g Tetraethoxysilan (TES 28 Wacker Chemie AG; 39 mmol) wurden zu einer Cyclodextrin haltigen Lösung aus 450 ml Wasser und 50 g gamma-Cyclodextrin (Wacker Chemie AG; 39 mmol) dosiert. Die nicht mischbaren Lösungen wurden bei Raumtemperatur in einem Horizontalschüttler in innigen Kontakt gebracht, wodurch sich ein weißer Niederschlag bildete. Der Niederschlag wurde sofort abgetrennt (Filtration oder Abzentrifugieren), mit Wasser nachgewaschen und im Trockenschrank bei 80°C getrocknet. Das erhaltene Pulver war bei trockener Lagerung bei Raumtemperatur stabil.
Beispiel 2: Analytik des Komplexes aus Cyclodextrin und Tetraethoxysilan
   Die Zusammensetzung des Komplexes aus Beispiel 1 wurde nach Lösen in Dimethylsulfoxid (DMSO)-d6 durch Integration der Signale der Ethylgruppe (TES) und von Cyclodextrin mit 1H-NMR bestimmt. Dabei zeigte sich, dass Tetraethoxysilan mit gamma-Cyclodextrin einen stöchiometrischen 1:1 Komplex bildete.
   Die Komplexe waren thermisch stabil. Während sich freies Tetraethoxysilan bereits bei geringer Temperaturerhöhung zu verflüchtigen begann und bei ca. 160°C vollständig zerstört war, war der TES-gamma-Cyclodextrin Komplex in der Thermoanalyse bis ca. 250°C stabil und desintegrierte zusammen mit dem Cyclodextrin ab ca. 280°C.
Beispiel 3: Herstellung und Analytik eines Komplexes aus gamma-Cyclodextrin und Tetraisopropoxysilan
   6,6 g Tetra-iso-propoxysilan (Gelest, ABCR) wurden zu einer cyclodextrinhaltigen Lösung aus 400 ml Wasser und 80 g gamma-Cyclodextrin (Wacker Chemie AG) dosiert. Die nicht mischbaren Lösungen wurden bei Raumtemperatur in einem Horizontalschüttler in innigen Kontakt gebracht, wodurch sich ein weißer Niederschlag bildete. Der Niederschlag wurde sofort abgetrennt (Filtration oder Abzentrifugieren), mit Wasser nachgewaschen und im Trockenschrank bei 80°C getrocknet. Das mit 97 % Ausbeute erhaltene Pulver war bei trockener Lagerung bei Raumtemperatur stabil. Die Stöchiometrie des Komplexes (gamma-CD : Silan) betrug 2:1 (1H-NMR). Die thermische Stabilität entsprach den Komplexen mit TEOS (Bsp. 1)
Beispiel 4: Herstellung und Analytik eines Komplexes aus Cyclodextrin und Tetra-n-propoxysilan
   13,2 g Tetra-n-propoxysilan (Gelest, ABCR) wurden zu einer cyclodextrinhaltigen Lösung aus 400 ml Wasser und 80 g gamma-Cyclodextrin (Wacker Chemie AG) dosiert. Die nicht mischbaren Lösungen wurden bei Raumtemperatur in einem Horizontalschüttler in innigen Kontakt gebracht, wodurch sich ein weißer Niederschlag bildete. Der Niederschlag wurde sofort abgetrennt (Filtration oder Abzentrifugieren), mit Wasser nachgewaschen und im Trockenschrank bei 80°C getrocknet. Das mit 91 % Ausbeute erhaltene Pulver war bei trockener Lagerung bei Raumtemperatur stabil. Die Stöchiometrie des Komplexes betrug 1:1 (1H-NMR). Die thermische Stabilität entsprach den Komplexen mit TEOS (Bsp. 1).
Beispiel 5: Freisetzung von Alkoholen aus Komplexen von gamma-Cyclodextrin und Alkoxysilanen
   Werden die in den Beispiel 1, 3, und 4 hergestellten pulverförmigen Komplexe hydrolytischen Bedingungen (Feuchtigkeit, Wasserzutritt) ausgesetzt, kann das im Komplex gebundene Alkoxysilan die gebundenen Alkohole langsam abspalten. Endprodukte dieser Reaktion sind SiO₂ und die entsprechenden Alkohole.
   Die Hydrolyse konnte mittels 1H-NMR in D₂O verfolgt werden, da frei vorliegende Alkohole (Ethanol, n-Propanol, iso-Propanol) und Silan gebundene Alkoholreste gut getrennte Signale im 1H-NMR zeigten.
Beispiel 6: Verwendung von gamma-Cyclodextrin - Alkoxysilan Komplexen als Biocid
   Alkohole wie Ethanol, n-Propanol oder iso-Propanol sind effektive und verbreitete Verbindungen, um unerwünschte Keime abzutöten bzw. zu kontrollieren. Cyclodextrin-Alkoxysilan Komplexe sind in der Lage, Alkohol freizusetzen, gamma-Cyclodextrin - Alkoxysilan Komplexe können daher als eine pulverförmige Formulierung von den entsprechenden Alkoholen angesehen werden. Bei Kontakt mit Feuchtigkeit kann der Alkohol freigesetzt werden und seine biocide Wirkung entfalten.
   Die Versuche wurden in Suspension mit verschiedenen Keimen (Bakterien, Pilze) durchgeführt:
   Staphylococcus (S.) epidermidis ATCC 12228
   Micrococcus (M.) sedentarius DSM 20317
   Trichophyton (T.) rubrum ATCC 28189

Die Tests erfolgten in Anlehnung an die Richtlinie der DGHM (Deutsche Gesellschaft für Hygiene und Mikrobiologie), bewertet wurde das Keimwachstum (Medien entsprechend den Angaben von ATCC bzw. DSM) nach einer Einwirkzeit von 3 Tagen. Die Prüfkonzentration lag bei 30 %. Die Keime wurden bei 37°C kultiviert.

Das Wachstum der Organismen wurde am Ausmaß der Trübung des Mediums bewertet. In allen Fällen von Trübung (resp. Wachstum) wurden Subkulturen zum Zweck der Reidentifizierung der jeweils verwendeten Testkeime nach laborüblichen Differenzierungsmethoden durchgeführt.

In der Tabelle sind die Ergebnisse der Tests für die drei Silankomplexe (gamma-CD mit Tetraethoxy - TEOS, Tetra-n-Proxy - T-n-Pr und Tetraisopropoxy - T-iso-Pr zusammengestellt.
+ bedeutet: Wachstum in Gegenwart des hydrolysierenden Silankomplexes
- bedeutet: keine Wachstum in Gegenwart des hydrolysierenden Silankomplexes

| | TEOS | T-n-Pr | T-iso-Pr |
|---|---|---|---|
| Staphylococcus (S.) epidermidis ATCC 12228 | - | - | + |
| Micrococcus (M.) sedentarius DSM 20317 | + | - | + |
| Trichophyton (T.) rubrum ATCC 28189 | + | + | - |

Die gamma-Cyclodextrin - Alkoxysilan Komplexe sind in der Lage, bei Kontakt mit Wasser verschiedene Keime am Wachstum zu hemmen.

## Patentansprüche

1. Isolierter Komplex aus einem Cyclodextrin oder Cyclodextrinderivat und einem Silan **dadurch gekennzeichnet,**
**dass** das Silan eine bei 20°C und Normdruck flüssige niedermolekulare Siliziumverbindung der allgemeinen Formel (I)
YₑSiR¹₍₄₋ₑ₎ (I)
ist, wobei Y Wasserstoff, eine OH-Gruppe, Halogen, eine OR¹-Gruppe bedeutet,
R¹ einen gegebenenfalls Heteroatom-substituierten aliphatischen oder aromatischen Kohlenwasserstoffrest mit 1-12 Kohlenstoffatomen bedeutet und
e die Werte 1, 2, 3 und 4 annehmen kann.

2. Komplex gemäß Anspruch 1, **dadurch gekennzeichnet, dass** das Silan ein hydrolyseempfindliches Silan ist.

3. Komplex gemäß Anspruch 1 oder 2, **dadurch gekennzeichnet, dass** das Silan ein Alkoxysilan ist.

4. Komplex gemäß einem der Ansprüche 1 bis 3, **dadurch gekennzeichnet, dass** das Cyclodextrin bzw. Cyclodextrinderivat eine Verbindung der Formel (II) 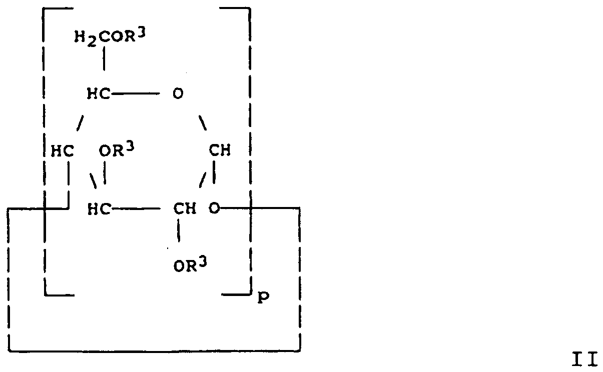 wobei R³ gleich oder verschieden sein kann und Wasserstoff oder einen gegebenenfalls Heteroatom-substituierten aliphatischen oder aromatischen Kohlenwasserstoffrest mit 1-12 Kohlenstoffatomen bedeutet und p ein ganzzahliger Wert von 6 bis 120 ist.

5. Komplex gemäß einem der Ansprüche 1 bis 4, **dadurch gekennzeichnet, dass** das Cyclodextrin bzw. Cyclodextrinderivat gamma-Cyclodextrin oder ein Derivat des gamma-Cyclodextrins ist.

6. Verfahren zur Herstellung eines Komplexes gemäß einem der Ansprüche 1 bis 5 **dadurch gekennzeichnet, dass** das Silan und das Cyclodextrin bzw Cyclodextrinderivat miteinander über einen Zeitraum von 1 min. bis 24 h in Kontakt gebracht werden und der gebildete Komplex anschließend sofort von einem ggf. vorhandenen Lösungsmittel abgetrennt wird.

7. Verfahren gemäß Anspruch 6 **dadurch gekennzeichnet, dass** Cyclodextrine bzw. Cyclodextrinderivate und Silane im Molverhältnis von 10:1 bis 1:10, bevorzugt 3:1 bis 1:3, besonders bevorzugt 1:1, eingesetzt werden.

8. Verfahren gemäß Anspruch 5 oder 6, **dadurch gekennzeichnet, dass** bei dem in Kontakt bringen ein Lösungsmittel ausgewählt aus der Gruppe Wasser, Alkohol, Aceton, Tetrahydrofuran Dimethylsulfoxid, Acetonitril, Chloroform, Diethylether, Ethylacetat, p-Xylol Alkane und deren Gemische vorhanden ist.

9. Verwendung der Komplexe gemäß Anspruch 1 bis 5 zur gezielten Freisetzung von Silanen oder von labilen Bestandteilen des komplexierten Silans.

## Claims

1. Isolated complex of a cyclodextrin or cyclodextrin derivative and a silane, **characterized in that** the silane is a low-molecular-weight silicon compound of the general formula (I)
YₑSiR¹₍₄₋ₑ₎ (I),
where Y represents hydrogen, an OH group, halogen, an OR¹ group,
R¹ represents an optionally hetero-atom-substituted aliphatic or aromatic hydrocarbon radical having 1-12 carbon atoms and
e can assume the values 1, 2, 3 and 4,
which compound is liquid at 20°C and standard pressure.

2. Complex according to Claim 1, **characterized in that** the silane is susceptible to hydrolysis.

3. Complex according to Claim 1 or 2, **characterized in that** the silane is an alkoxysilane.

4. Complex according to any of Claims 1 to 3, **characterized in that** the cyclodextrin or cyclodextrin derivative is a compound of the formula (II) 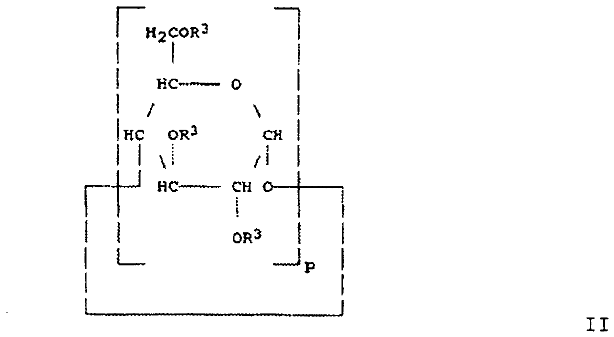 where R³ can be identical or different and represents hydrogen or an optionally hetero-atom-substituted aliphatic or aromatic hydrocarbon radical having 1-12 carbon atoms and p is an integer from 6 to 120.

5. Complex according to any of Claims 1 to 4, **characterized in that** the cyclodextrin or cyclodextrin derivative is gamma-cyclodextrin or a derivative of gamma-cyclodextrin.

6. Process for the preparation of a complex according to any of Claims 1 to 5, **characterized in that** the silane and the cyclodextrin or cyclodextrin derivative are brought into contact with each other over a period of from 1 min to 24 h and the complex formed is subsequently immediately separated from any solvent which may be present.

7. Process according to Claim 6, **characterized in that** cyclodextrins or cyclodextrin derivatives and silanes are employed in a molar ratio of from 10:1 to 1:10, preferably 3:1 to 1:3, especially preferably 1:1.

8. Process according to Claim 5 or 6, **characterized in that** a solvent selected from the group consisting of water, alcohol, acetone,
tetrahydrofuran, dimethyl sulfoxide, acetonitrile, chloroform, diethyl ether, ethyl acetate, p-xylene, alkanes and their mixtures is present during the bringing-into-contact.

9. Use of the complexes according to any of Claims 1 to 5 for the targeted release of silanes or unstable components of the complexed silane.

## Revendications

1. Complexe isolé d'une cyclodextrine ou d'un dérivé de cyclodextrine et d'un silane, **caractérisé en ce que** le silane est un composé silicié de bas poids moléculaire, liquide à 20°C et à la pression normale, de formule générale (I)
YₑSiR¹₍₄₋ₑ₎ (I)
où
Y signifie hydrogène, un groupe OH, halogène, un
groupe OR¹,
R¹ signifie un radical hydrocarboné aliphatique ou
aromatique, le cas échéant substitué par un hétéroatome, comprenant 1 à 12 atomes de carbone, et
e peut valoir 1, 2, 3 et 4.

2. Complexe selon la revendication 1, **caractérisé en ce que** le silane est un silane sensible à l'hydrolyse.

3. Complexe selon la revendication 1 ou 2, **caractérisé en ce que** le silane est un alcoxysilane.

4. Complexe selon l'une quelconque des revendications 1 à 3, **caractérisé en ce que** la cyclodextrine ou, selon le cas, le dérivé de cyclodextrine est un composé de formule (II) 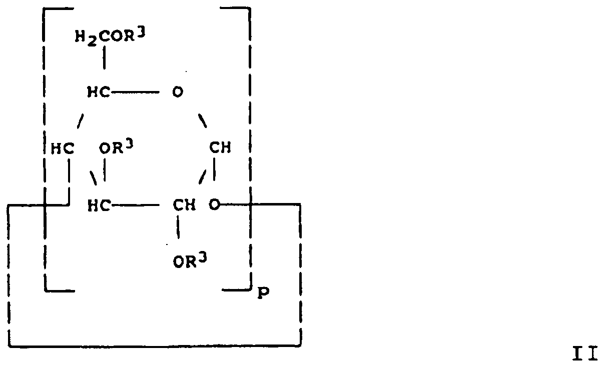 où R³ peut être identique ou différent et signifie hydrogène ou un radical hydrocarboné aliphatique ou aromatique, le cas échéant substitué par un hétéroatome, comprenant 1 à 12 atomes de carbone, et p vaut un nombre entier de 6 à 120.

5. Complexe selon l'une quelconque des revendications 1 à 4, **caractérisé en ce que** la cyclodextrine ou, selon le cas, le dérivé de cyclodextrine est la gamma-cyclodextrine ou un dérivé de la gamma-cyclodextrine.

6. Procédé pour la préparation d'un complexe selon l'une quelconque des revendications 1 à 5, **caractérisé en ce que** le silane et la cyclodextrine ou, selon le cas, le dérivé de cyclodextrine sont mis en contact l'un avec l'autre pendant un laps de temps de 1 min à 24 h et le complexe formé est ensuite immédiatement séparé d'un solvant le cas échéant présent.

7. Procédé selon la revendication 6, **caractérisé en ce que** les cyclodextrines ou, selon le cas, les dérivés de cyclodextrine et les silanes sont utilisés dans un rapport molaire de 10:1 à 1:10, de préférence de 3:1 à 1:3, de manière particulièrement préférée de 1:1.

8. Procédé selon la revendication 5 ou 6, **caractérisé en ce que** lors de la mise en contact, un solvant, choisi dans le groupe formé par l'eau, un alcool, l'acétone, le tétrahydrofuranne, le diméthylsulfoxyde, l'acétonitrile, le chloroforme, le diéthyléther, l'acétate d'éthyle, le p-xylène, les alcanes et leurs mélanges, est présent.

9. Utilisation des complexes selon les revendications 1 à 5 pour la libération ciblée de silanes ou de constituants labiles du silane complexé.
